# FASCICULE DE BREVET EUROPEEN

(11) **EP 2 495 013 B1**
(45) Date de publication et mention de la délivrance du brevet: **24.07.2013**
(21) Numéro de dépôt: 12153639.5
(22) Date de dépôt: 02.02.2012
(51) Int. Cl.: A61N 1/362

(54) **Ensemble pour la recherche d'une configuration optimale d'un implant de resynchronisation cardiaque bi-, tri- ou multiventriculaire**
Anordnung für die Suche nach einer optimalen Konfiguration eines Implantats zur bi-, tri- oder multiventrikulären Resynchronisation des Herzens
Assembly for searching for an optimal configuration of a bi-, tri- or multi-ventricular cardiac resynchronisation implant

(30) Priorité: 03.03.2011 FR 1151729
(43) Date de publication de la demande: 05.09.2012
(73) Titulaire: Sorin CRM SAS, 92140 Clamart Cedex (FR)
(72) Inventeur: Renesto, Fabrizio, 10013 Borgofranco d'Ivrea (TO) (IT); Giorgis, Lionel, 22000 Saint Brieuc (FR)
(74) Mandataire: Dupuis-Latour, Dominique

(56) Documents cités:
- EP-A1- 1 736 203
- WO-A1-2006/049538

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer au coeur, en tant que de besoin, des impulsions électriques permettant d'assurer une stimulation conjointe et permanente des ventricules gauche et droit afin de resynchroniser ces derniers, technique dite "CRT" (*Cardiac Resynchronization Therapy*) ou "BVP" (*Bi-Ventricular Pacing*).

À cet effet, on implante au patient un appareil et des sondes munies d'électrodes permettant de stimuler en divers sites l'un et l'autre ventricule. Le dispositif applique entre les instants respectifs de stimulation des ventricules gauche et droit un délai dit "délai interventriculaire" (DVV ou VVD) ajusté de manière à resynchroniser la contraction des deux ventricules pour optimiser l'état hémodynamique du patient.

Un stimulateur CRT est par exemple divulgué dans le EP 1 108 446 A1 (ELA Medical), qui décrit un dispositif permettant d'appliquer entre les deux stimulations ventriculaires un DVV variable, ajusté de manière à resynchroniser la contraction des ventricules avec optimisation fine de l'état hémodynamique du patient. Le DVV peut être nul, positif (le ventricule gauche étant stimulé après le ventricule droit), ou négatif (le ventricule droit étant stimulé après le ventricule gauche).

Des études cliniques ont permis d'observer des résultats souvent spectaculaires pour des patients en insuffisance cardiaque non améliorée par les traitements classiques, dès lors que les paramètres de la thérapie CRT ont été ajustés de façon précise en fonction du patient et de la nature de son trouble.

Mais l'implantation de ces dispositifs représente pour le praticien une intervention très délicate, car il doit effectuer à ce moment de nombreux choix.

*En premier lieu*, il convient de déterminer pour chacune des sondes le meilleur site de stimulation.

On appellera "sites de stimulation" les emplacements physiques des électrodes de stimulation par rapport au tissu du myocarde ; généralement, ces sites ne peuvent être choisis qu'au moment de l'implantation, par un positionnement approprié des électrodes. Il est important de vérifier l'efficacité des sites choisis, en raison de l'influence possible à long terme de l'efficacité de la thérapie de resynchronisation. Dans certains cas, le dispositif multisite comporte plusieurs électrodes placées dans une même cavité, et une modification du ou des sites de stimulation dans cette cavité est alors possible par des commutations internes du dispositif.

En tout état de cause, lors de l'intervention, le praticien teste plusieurs sites possibles par des repositionnements successifs de la sonde, jusqu'à trouver celui qui, selon lui, est le plus approprié.

Un autre aspect du développement de ces appareils est la multiplication du nombre d'électrodes, notamment pour les appareils dits "multisite" qui permettent de choisir les sites de stimulation/détection optimisant le fonctionnement de l'appareil.

La multiplication des électrodes peut également résulter de la présence à un même niveau de la sonde de plusieurs électrodes sectorielles (électrodes dirigées spécifiquement dans une direction radiale par rapport à la sonde, au site de stimulation), avec possibilité de sélectionner l'une ou l'autre de ces électrodes sectorielles afin d'optimiser la délivrance des impulsions au site choisi. Il en est ainsi notamment des sondes implantées dans le réseau coronaire veineux, destinées à la stimulation indirecte d'une cavité gauche : avec plusieurs électrodes sectorielles, il est possible de sélectionner celle qui est tournée vers la paroi de l'épicarde, face à la cavité et en contact avec cette paroi.

*En second lieu*, avec les dispositifs de nouvelle génération permettant une stimulation sur plus de deux sites ventriculaires, il convient de déterminer si ce mode de stimulation "triventriculaire" ou "multiventriculaire" est ou non préférable à un mode biventriculaire conventionnel.

Ainsi, le praticien peut se trouver confronté à un choix entre un mode de stimulation biventriculaire standard (ventricules droit et gauche), triventriculaire (stimulation simultanée par trois électrodes, avec une électrode additionnelle à droite ou à gauche), ou même multiventriculaire (avec des sondes multi-électrodes dont plusieurs électrodes de la même sonde sont utilisées concurremment). Par des commutations appropriées le praticien pourra choisir le mode de stimulation le plus approprié, mais le nombre de configurations possibles augmente très rapidement avec la multiplication des électrodes, rendant d'autant plus délicate la tâche à effectuer par le praticien, confronté à un choix entre un nombre important de configurations différentes.

*En troisième lieu*, il convient de paramétrer le dispositif de façon appropriée, notamment les délais interventriculaire DVV et atrioventriculaire DAV.

Les multiples possibilités résultant de ces divers choix seront appelées par la suite "configurations de stimulation".

Le but de l'invention est de proposer un ensemble ou équipement qui puisse aider le praticien à trouver la configuration de stimulation la plus appropriée, notamment pour lui permettre d'évaluer les modifications d'efficacité des configurations de stimulation suite à des repositionnements de sonde ainsi que, le cas échéant, des sélections de sondes additionnelles (stimulation biventriculaire, triventriculaire ou multiventriculaire) ou des commutations d'électrodes dans le cas d'une sonde multi-électrodes.

Par ailleurs, il peut être nécessaire de réévaluer ultérieurement ces choix, pour éventuellement réajuster les réglages. En effet, les effets bénéfiques procurés par la thérapie CRT peuvent conduire, à terme, à modifier la configuration primitive et le paramétrage de la stimulation.

Le but premier de la présente invention est de définir des indices relatifs aux performances cardiaques hémodynamiques du patient, permettant d'optimiser le positionnement des sondes et le choix des configurations de stimulateur durant la procédure d'implantation, ainsi que la reprogrammation éventuelle des paramètres après implantation du dispositif CRT.

On constate en effet qu'aujourd'hui, même avec une mise en oeuvre complète des procédures, il reste environ 30 % de patients qui ne répondent pas à la thérapie CRT, avec les conséquences graves que l'on peut imaginer en termes de qualité de vie, d'hospitalisations liées à l'insuffisance cardiaque et de réduction de l'espérance de vie.

La plupart des études se concentrent aujourd'hui sur la manière de traiter cette population de patients réfractaires par expérimentation de nouvelles configurations de stimulation, et en cherchant à optimiser le paramétrage de la stimulation, aussi bien lors de l'implantation que de façon suivie, par des réévaluations périodiques.

Il existe de ce fait un besoin réel d'une technique permettant d'évaluer de façon simple, rapide, automatisée et précise l'incidence du choix des sites de stimulation et des différents paramètres de la thérapie CRT, notamment des délais DAV et DVV, de manière à pouvoir optimiser l'état hémodynamique du patient.

La technique de référence pour l'ajustement des paramètres de stimulation CRT est l'évaluation par échocardiographie avec estimation des délais caractéristiques de la systole, en particulier le temps d'ouverture de la valve aortique.

Cette procédure, qui doit être mise en oeuvre en milieu hospitalier et par un personnel qualifié, est longue et coûteuse et ne peut pas être appliquée aussi souvent que cela serait utile ou nécessaire. En outre, il n'est pas facile de pratiquer des mesures échographiques durant la procédure d'implantation, car le champ stérile ne permet pas un accès aisé au thorax du patient avec la sonde d'échographie.

D'autres techniques ont été proposées pour évaluer l'efficacité du choix des sites de stimulation et du paramétrage de la thérapie CRT.

Ainsi, le EP 1 736 203 A1 (ELA Medical) décrit un dispositif CRT qui utilise à cet effet les paramètres liés à l'accélération endocardiaque (ci-après désignée "EA") pour déterminer une configuration de stimulation optimale, au moment de l'implantation ou ultérieurement.

En effet, diverses études cliniques ont montré que l'accélération endocardiaque est un paramètre qui reflète très précisément et en temps réel les phénomènes liés aux mouvements de la cavité cardiaque, et permet de ce fait de fournir des informations très complètes sur la mécanique cardiaque, aussi bien dans le cas d'un fonctionnement normal que d'un fonctionnement déficient. L'accélération endocardiaque est par exemple mesurée par un accéléromètre intégré dans une sonde endocavitaire, comme cela est par exemple décrit dans le EP 0 515 319 A1 (Sorin Biomedica Cardio SpA).

Ce document propose d'évaluer l'efficacité de la stimulation sur la base d'un "indice de performance" établi à partir de paramètres issus d'un signal délivré par le capteur d'accélération endocardiaque, notamment : l'amplitude du premier pic d'accélération endocardiaque, la durée de ce pic, et la durée de la systole (intervalle séparant le premier et le second pic d'accélération endocardiaque).

Le WO 2006/049538 A1 (Sainte Jude Medical AB) décrit une technique consistant à évaluer un paramètre physiologique reflétant la performance hémodynamique du coeur pour une configuration de stimulation donnée, à partir de divers capteurs (pression, accélération, acoustique) placés sur une ou plusieurs sondes dont certaines peuvent être repositionnées ; de ce fait le signal délivré par ces capteurs va dépendre de la position courante de la sonde et ne peut constituer une référence fiable. La technique proposée comporte cependant un certain nombre d'inconvénients, notamment une optimisation basée sur l'analyse d'un unique paramètre physiologique (débit cardiaque, volume d'éjection, etc.). Or, certains patients peuvent être plus ou moins sensibles à l'un ou l'autre de ces paramètres, qui n'est pas toujours le même d'un patient à l'autre du fait de la réponse propre du patient, de sa pathologie et de l'évolution de celle-ci. Le paramètre analysé n'est donc pas nécessairement le plus pertinent par rapport aux modifications de la configuration de stimulation.

Le but de l'invention est de proposer une technique d'évaluation de la configuration de stimulation présentant une sensibilité et une spécificité accrues par rapport à ce qui a pu être proposé jusqu'à présent, notamment par le EP 1 736 203 A1 précité, en particulier à l'égard des changements de position des électrodes de stimulation.

Un autre but de l'invention est de proposer un équipement complet, mis à la disposition du praticien implanteur, qui soit spécifiquement adapté à la sélection des sites de stimulation lors de la procédure de mise en place des sondes et d'implantation d'un dispositif biventriculaire, triventriculaire ou multiventriculaire.

A cet effet, l'invention propose un ensemble pour la recherche d'une configuration optimale de stimulation au moyen d'un dispositif médical actif (prothèse cardiaque implantable ou dispositif externe utilisé temporairement) de resynchronisation par stimulation bi-, tri- ou multiventriculaire, cet ensemble comprenant, comme enseigné par le EP 1 736 203 A1 précité :
- des moyens de stimulation ventriculaire, aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante prédéterminée et modifiable ;
- un capteur d'accélération apte à délivrer un signal d'accélération endocardiaque EA représentatif des contractions et relaxations cycliques du coeur ; et
- des moyens aptes à isoler et prétraiter, dans le signal EA considéré sur un cycle cardiaque compris entre deux événements ventriculaires successifs : (i) une composante EA1 correspondant au premier pic d'accélération endocardiaque, associé à la contraction ventriculaire isovolumique, et (ii) une composante EA2 correspondant au second pic d'accélération endocardiaque, associé à la relaxation ventriculaire isovolumique.

Cet ensemble comprend en outre des moyens évaluateurs de l'efficacité de ladite configuration de stimulation courante, comprenant :
- des moyens pour extraire au moins deux paramètres des composantes EA1 et EA2 isolées et prétraitées :
   - des moyens pour combiner lesdits au moins deux paramètres en un indice composite représentatif de l'efficacité de ladite configuration de stimulation courante ;
   - des moyens pour déterminer une pluralité de valeurs dudit indice composite pour une pluralité correspondante de configurations de stimulation différentes ; et
   - des moyens pour déterminer une configuration de stimulation préférentielle à partir de ladite pluralité de valeurs dudit indice composite, par recherche d'un optimum dudit indice composite.

De façon caractéristique de l'invention, lesdits au moins deux paramètres caractéristiques des composantes EA1 et EA2 sont des paramètres du groupe formé par :
- *PEA1* = valeur de l'amplitude crête-à-crête de la composante EA1 ;
- *TstEA1* = durée d'apparition du début de la composante EA1, représentée par l'intervalle de temps séparant i) un marqueur temporel de début de cycle cardiaque et ii) le franchissement d'un seuil d'enveloppe d'énergie de la composante EA1 ;
- *LargEA1* = intervalle de temps séparant i) ledit franchissement du seuil d'enveloppe d'énergie de la composante EA1 de ii) l'instant du pic de ladite enveloppe d'énergie de la composante EA1; et
- *Syst* = durée de la systole, représentée par l'intervalle de temps séparant le début de la composante EA1 du début de la composante EA2,

L'indice composite est un indice du groupe formé par :
- *Ind1* = *(TstEA 1 x LargEA1)* / *(Syst x PEA1) ;*
- *Ind2* = *(TstEA1* x *LargEA1)* / *[(Syst - LargEA1)* x *PEA1] ;*
- *Ind3* = *(TstEA1 x LargEA1)* / *(Syst)* ; et
- *Ind4* = *(TstEA1)* / *(PEA1)*,
ledit optimum étant un minimum de cet indice.

L'ensemble comprend avantageusement une sonde auriculaire portant le capteur d'accélération, et des sondes ventriculaires droite et gauche portant à leur extrémité distale des électrodes aptes à définir respectivement les sites de stimulation ventriculaire droit(s) et gauche(s) selon la configuration de stimulation courante. Il peut également comprendre une sonde ventriculaire additionnelle, droite ou gauche, portant à son extrémité distale des électrodes aptes à définir au moins un site de stimulation ventriculaire additionnel, définissant la configuration de stimulation courante en combinaison avec les sites de stimulation ventriculaire droit(s) et gauche(s).

L'invention porte également sur un ensemble complet comprenant :
- la prothèse cardiaque implantable de resynchronisation par stimulation bi-, tri- ou multiventriculaire ;
- une sonde auriculaire portant le capteur d'accélération ;
- des sondes ventriculaires droite et gauche portant à leur extrémité distale des électrodes aptes à définir respectivement les sites de stimulation ventriculaire droit(s) et gauche(s) selon la configuration de stimulation courante ;
- éventuellement une sonde ventriculaire additionnelle, droite ou gauche, portant à son extrémité distale des électrodes aptes à définir au moins un site de stimulation ventriculaire additionnel, définissant la configuration de stimulation courante en combinaison avec les sites de stimulation ventriculaire droit(s) et gauche(s) ;
- un équipement externe de détection/stimulation ;
- un boîtier d'interfaçage comprenant des moyens de traitement dudit signal d'accélération endocardiaque EA, ainsi que des moyens pour coupler l'équipement externe aux sondes auriculaire et ventriculaires ; et
- des moyens programmateurs externes, couplés au boîtier d'interfaçage pour recevoir le signal d'accélération endocardiaque EA traité, et comprenant les moyens évaluateurs de l'efficacité de la configuration de stimulation.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une série de trois chronogrammes illustrant différents signaux caractérisant l'activité cardiaque au cours d'un cycle donné.
La Figure 2 montre, de façon plus détaillée, l'allure du signal d'accélération endocardiaque au cours d'un cycle cardiaque donné, avec les différents paramètres utilisés pour la mise en oeuvre de l'invention.
La Figure 3 présente schématiquement les divers éléments, sous forme de blocs fonctionnels, de l'équipement mis à la disposition du praticien au moment de l'implantation pour la sélection des sites de stimulation selon l'invention.
La Figure 4 illustre plus précisément le diagramme affiché par l'interface de sélection des sites de stimulation.
La Figure 5 est un organigramme décrivant les diverses étapes successives du procédé de mise en oeuvre de l'ensemble de l'invention.
La Figure 6 expose les diverses étapes d'un suivi du patient sur le long terme par analyse de l'évolution d'un ou plusieurs des indices calculés selon les enseignements de l'invention.

On va maintenant décrire un exemple de réalisation de l'invention, en référence aux dessins annexés.

L'invention peut notamment être appliquée aux dispositifs implantables tels que ceux de la famille *Paradym CRT* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre certaines des fonctions de l'invention qui seront décrites ci-dessous (optimisation des paramètres et suivi de l'état du patient). L'adaptation de ces appareils à la mise en oeuvre des fonctions de l'invention est à la portée de l'homme du métier, et elle ne sera pas décrite en détail.

La technique de l'invention est basée sur l'analyse de l'accélération endocardiaque (ci-après désignée "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée dans l'oreillette ou le ventricule et intégrant un microaccéléromètre permettant de mesurer l'accélération endocardiaque.

On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boîtier d'un implant. L'invention est également applicable à l'analyse d'un signal EA externe recueilli de manière non invasive, par exemple issu d'un capteur fixé sur la poitrine du patient au niveau du sternum.

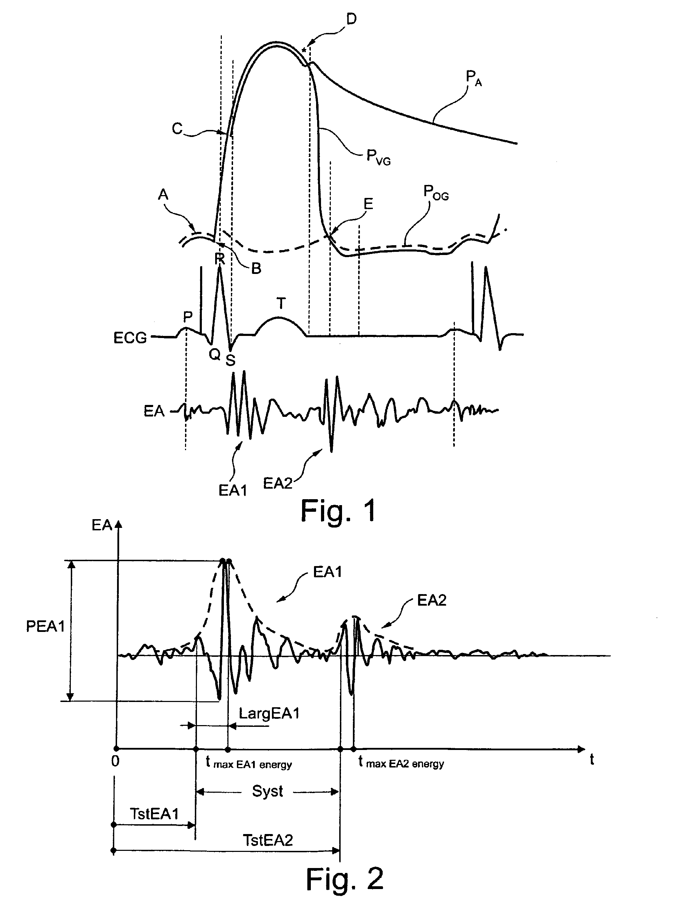

La Figure 1 illustre les différents signaux caractérisant l'activité du coeur au cours d'un cycle cardiaque, avec : le profil des pressions intracardiaques (P_{A}, P_{VG} et P_{OG}), un relevé d'électrocardiogramme de surface (ECG), et les variations du signal d'accélération endocardiaque (EA).

La caractéristique P_{A} illustre les variations de la pression aortique, P_{VG} celles du ventricule gauche et P_{OG} celles dans l'oreillette gauche. Les points A à E correspondent aux différentes phases suivantes : A contraction de l'oreillette gauche, B fermeture de la valvule mitrale, C ouverture de la valvule aortique, D fermeture de la valvule aortique, E ouverture de la valvule mitrale.

Le signal ECG présente successivement l'onde P correspondant à la dépolarisation des oreillettes, le complexe QRS correspondant à la dépolarisation des ventricules et l'onde T de repolarisation ventriculaire.

Le signal d'accélération endocardiaque EA recueilli au cours d'un cycle cardiaque donné (ci-après "signal EA") forme deux composantes principales, correspondant aux deux bruits majeurs du coeur (sons S1 et S2 du phonocardiogramme) qu'il est possible de reconnaître dans chaque cycle cardiaque :
- la composante EA1, débutant à la suite du complexe QRS, est engendrée par une combinaison de la fermeture des valves auriculoventriculaires, de l'ouverture des valves semi-lunaires et de la contraction du ventricule gauche. Les variations d'amplitude de cette composante EA1 sont étroitement liées aux variations de la pression dans le ventricule (l'amplitude maximale crête-à-crête étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ; et
- la composante EA2 survient pendant la phase de relaxation ventriculaire isovolumique. Elle accompagne la fin de la systole ventriculaire et est principalement produite par la fermeture des valves aortiques et pulmonaires.

Pour la mise en oeuvre de la présente invention, il convient tout d'abord d'extraire du signal EA, et plus précisément des deux composantes EA1 et EA2, des caractéristiques corrélées à des intervalles de temps de la systole et à d'autres indices de performance hémodynamique du myocarde, par un traitement spécifique de ce signal EA.

Le traitement préliminaire des composantes EA implique, tout d'abord, d'individualiser les cycles cardiaques successifs dans le signal EA recueilli en continu, en déterminant des marqueurs de début de cycle permettant de séparer ces cycles et d'isoler une série de sous-signaux EA bornés dans le temps correspondant chacun à une durée d'un seul cycle cardiaque.

Dans le cas d'un signal EA endocavitaire, les marqueurs temporels de début de cycle peuvent être fournis par l'implant lui-même ou par un dispositif externe lors de l'implantation, qui selon le mode de fonctionnement garde en mémoire les instants de la stimulation V, ou bien les instants de détection de l'onde R. Le marqueur temporel de début de cycle, qui constitue l'origine des temps, sera désigné "O".

L'étape suivante consiste à isoler les composantes EA1 et EA2 dans chacun des sous-signaux bornés dans le temps correspondant à un cycle cardiaque. Chacune de ces composantes EA1 et EA2 sera représentée par un ensemble de valeurs successives décrivant la variation continue du signal EA dans une fenêtre temporelle donnée s'étendant autour du pic atteint par le signal EA (pic PEA1 ou pic PEA2), sur une fraction de la durée d'un cycle cardiaque. Concrètement, chaque composante sera constituée d'un sous-ensemble des échantillons du signal EA obtenus après numérisation de ce signal sur la durée du cycle cardiaque.

Chacune de ces composantes représente donc une fraction du signal EA sur la durée d'un cycle cardiaque, chaque cycle cardiaque comprenant une pluralité de composantes différentes qui se succéderont, notamment les deux premières composantes EA1 et EA2, qui sont suivies des composantes secondaires EA3 et EA4.

De préférence, les composantes EA1 et EA2 du signal EA sont déterminées avec un moyennage sur plusieurs cycles, typiquement trois à cinq cycles, en appliquant une technique telle que celle exposée dans la demande EP 2 092 885 A1 (ELA Medical), technique qui permet notamment d'éliminer les variations cycle à cycle en recalant dans le temps les composantes successives avant de les moyenner.

Essentiellement, cette technique consiste à effectuer un prétraitement du signal EA recueilli en continu, avec :
- découpage du signal EA en sous-signaux correspondant chacun à la durée d'un cycle cardiaque et repérés par un marqueur de début de cycle permettant de réaliser ce découpage ;
- segmentation de chacun de ces sous-signaux de manière à individualiser les composantes EA1 et EA2 dans une fenêtre temporelle donnée ;
- pour la composante courante EA1 ou EA2 ainsi isolée sur un cycle, recherche d'un pic d'intercorrélation par rapport aux composantes EA1 (ou EA2) des autres cycles recueillis ;
- calcul d'un décalage temporel correspondant ; et
- application du décalage temporel ainsi calculé à la composante courante, de manière à aligner celle-ci par rapport aux autres.

Les traitements d'analyse pourront être ensuite exécutés sur ces composantes EA1 et EA2 successives, avec élimination du biais de la variabilité cycle à cycle grâce à ce prétraitement.

L'invention est basée sur l'extraction et l'analyse d'un certain nombre de paramètres caractéristiques des composantes EA1 et EA2, paramètres qui seront ensuite combinés pour donner des indices composites que le praticien cherchera à optimiser (minimiser ou maximiser, selon le cas), l'optimum atteint reflétant la meilleure configuration de stimulation parmi toutes celles qui auront pu être testées.

Ces paramètres sont au nombre de quatre, et sont illustrés sur la Figure 2 qui représente la variation du signal EA au cours d'un cycle cardiaque, avec ses deux composantes EA1 et EA2 :
- l'amplitude crête-à-crête de la composante EA1, désignée *PEA1* ;
- le temps d'apparition du début de la composante EA1, notée *TstEA1*, qui est une durée comptée à partir de l'origine des temps O sur la Figure 2 ;
- un indicateur de la durée de la composante EA1, désigné *LargEA1*, qui est l'intervalle de temps séparant *TstEA1* de l'instant *t_{maxEA1energy}* du pic d'énergie de la composante EA1 ; et
- la durée de la systole, désignée *Syst*, correspondant à l'intervalle de temps séparant *TstEA1*, qui marque le début de la composante EA1, de *TstEA2*, qui marque le début de la composante EA2.

Les temps *TstEA1* et *TstEA2* de début des composantes EA1 et EA2 peuvent être obtenus par exemple en seuillant une enveloppe d'énergie obtenue en élevant au carré la valeur des échantillons de signal (enveloppe illustrée en tiretés sur la Figure 2), puis en appliquant une fenêtre de lissage, de 100 ms par exemple. Les temps *TstEA1* et *TstEA2* correspondront au franchissement d'un seuil qui peut être par exemple 10 % du maximum de l'énergie sur la fenêtre considérée correspondant à l'une et l'autre des deux composantes EA1 et EA2.

Les temps respectifs d'occurrence de l'énergie maximale des composantes EA1 et EA2, notés *t_{maxEA1energy}* et *t_{maxEA2energy}*, sont utilisés pour calculer *LargEA1* = *t_{maxEA1energy}* - *TStEA1.*

Cette méthode de détermination des instants caractéristiques des composantes EA1 et EA2 est décrite ainsi que d'autres dans le EP 2 092 885 A1 précité, auquel on pourra se référer pour plus de détails.

Les paramètres ci-dessus ont été choisis pour les raisons suivantes :
- l'amplitude *PEA1* est associée à la contractilité cardiaque (des études cliniques ont montré une corrélation forte avec le maximum de LVdP/dT). Une stimulation efficace, pour une position optimale de l'électrode de stimulation, a pour effet de maximiser ce paramètre ;
- le temps *TstEA1* d'apparition du début de la composante EA1 est associé à la phase de contraction isovolumique du cycle cardiaque, et en particulier au temps d'ouverture de la valve aortique, durée également désignée "intervalle de pré-éjection gauche" LPEI. Les études cliniques semblent montrer qu'une minimisation de ce paramètre reflète une amélioration de la resynchronisation ventriculaire et des performances hémodynamiques ; on constate également que ce paramètre est également très sensible aux modifications de position des électrodes de la sonde ;
- la durée *LargEA1* est un bon indicateur de la resynchronisation. Il convient de rechercher pour cette durée des valeurs réduites, et il a été également constaté que ce paramètre est beaucoup plus sensible aux modifications des positions des électrodes que, notamment, la durée complète de la composante EA1 (intervalle de temps entre le début et la fin de la composante EA1). Il est également plus fiable par rapport à une technique consistant à utiliser l'instant de fin de la composante EA en détectant celui-ci par seuillage et moyennage sur plusieurs cycles: on constate alors une variabilité relativement élevée par rapport à l'instant où est atteint le pic ;
- la durée *Syst* de la systole est associée à la durée séparant la fermeture de la valve mitrale et celle de la valve aortique, et ce paramètre donne donc une bonne indication des performances de la phase d'éjection ; ce paramètre est également très sensible aux modifications de position des électrodes.

En d'autres termes, les quatre paramètres ci-dessus présentent une sensibilité et une précision particulièrement élevées en réponse aux modifications des configurations de stimulation, ce qui permettra de guider de façon très efficace le praticien pour le choix et l'ajustement des sites de stimulation.

Ces paramètres sont également sensibles au paramétrage du stimulateur (notamment les délais DAV et DVV), et pourront être utilisés pour le réglage initial ou le réajustement ultérieur.

Toutefois, l'optimisation d'un seul des quatre paramètres ci-dessus n'est pas toujours suffisante pour déterminer de façon certaine la meilleure configuration de stimulation et les meilleurs paramétrages du dispositif. L'utilisation d'un seul paramètre conduirait en effet à un nombre trop important de déterminations erronées, et c'est pourquoi l'invention propose de combiner une pluralité de ces paramètres (au moins deux) en un ou plusieurs indices dits "indices composites" pour décider de façon fiable du meilleur choix de la configuration de stimulation.

Les indices composites suivants ont été choisis dans la mesure où ils sont tous très sensibles aux changements de position des sites de stimulation :
- *Ind1 =* (*TstEA1 x LargEA1*) / (*Syst x PEA1*) : cet indice combine les diverses durées caractéristiques du cycle cardiaque et les paramètres reflétant la contractilité, et présente la sensibilité la plus élevée aux modifications de la position des sites de stimulation, et la meilleure corrélation avec les références cliniques ;
- *Ind2 =* (*TstEA1 x LargEA1*) / [(*Syst- LargEA1*) *x PEA1*] : cet indice est très semblable au précédent, mais la différence *Syst - LargEA1* permet de rendre plus stable le facteur relatif à la durée de la systole ;
- *Ind3 =* (*TstEA1 x LargEA1*) / (*Syst*) : cet indice est dérivé du premier, sans contribution de la contractilité, et il est plutôt destiné aux applications où l'optimisation est liée de façon stricte aux durées du cycle cardiaque et ne dépendant pas, ou peu, de la contractilité ;
- *Ind4* = (*TstEA1*) / (*PEA1*) : cet indice est un indice subsidiaire ; il reflète les performances cardiaques lorsqu'il s'agit d'optimiser uniquement celles concernant la phase de contraction ventriculaire. Il peut être utilisé notamment si la fermeture de la valve aortique est difficile à détecter (c'est-à-dire quand la composante EA2 est peu visible), conduisant à une détermination incertaine de la durée de la systole *Syst*, du fait d'un signal peu fiable lié à la phase de relaxation du cycle cardiaque.

Pour tous ces indices, l'optimum à rechercher sera le minimum.

Les indices composites ci-dessus se sont révélés très efficaces lors d'études cliniques, et adaptés à de nombreuses conditions hémodynamiques différentes de patients, pour des pathologies variées. Cette liste n'est cependant pas limitative, et d'autres combinaisons des paramètres indiqués plus haut peuvent être utilisées dans d'autres situations ou pathologies particulières.

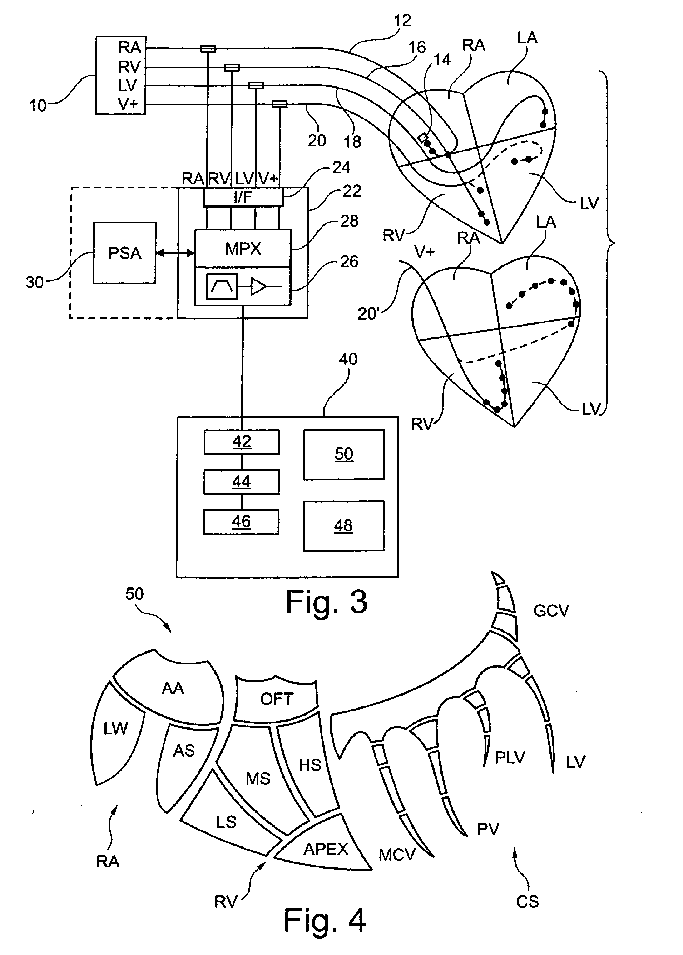

On va maintenant décrire la manière de réaliser, concrètement, cette optimisation au cours de la phase d'implantation, en référence aux Figures 3 à 5.

La Figure 3 illustre de façon schématique l'installation utilisée pour l'optimisation de la position des sites de stimulation et des configurations de paramètres programmés.

L'ensemble comprend un implant de type stimulateur et/ou resynchroniseur 10, pourvu d'une pluralité de sondes implantées dans le coeur, avec une sonde auriculaire droite RA, référencée 12, équipée à son extrémité distale d'un accéléromètre 14 capable de délivrer un signal d'accélération endocardiaque. On notera que le capteur EA est disposé ici sur une sonde auriculaire, qui est de ce fait une sonde autre que celles qui seront déplacées pour rechercher une configuration de stimulation optimale ; en d'autres termes, la sonde sur laquelle est placé le capteur EA ne participe pas à la définition de la "configuration de stimulation" au sens indiqué plus haut.

Le dispositif est également relié à des sondes ventriculaire droite RV et ventriculaire gauche LV, référencées 16, 18, ainsi qu'éventuellement à une sonde ventriculaire additionnelle V+, référencée 20, pouvant être utilisée notamment pour améliorer les performances ventriculaires si une stimulation par les sondes RV et LV ne procure pas une amélioration suffisante de l'hémodynamique.

Ces sondes sont reliées au générateur 10 par l'intermédiaire de câbles stériles, qui sont par ailleurs pourvus d'une dérivation vers un boîtier d'interfaçage 22 destiné à la recherche de la configuration de stimulation optimale.

La sonde ventriculaire peut être également, comme illustré en 20' sur la variante en bas à droite, une sonde de type "multi-électrodes" ventriculaire droite ou gauche. Les EP 1 938 861 A1 et le EP 2 082 684 A1 (tous les deux au nom de ELA Medical) décrivent une telle sonde munie d'une pluralité d'électrodes au voisinage de son extrémité distale, par exemple dix électrodes, associées à une puce, encapsulée de manière hermétique au voisinage des électrodes dans un anneau rigide, assurant le multiplexage/démultiplexage des électrodes avec un bus commun formé par deux conducteurs isolés s'étendant sur toute la longueur de la sonde jusqu'au connecteur proximal permettant le couplage avec le générateur, ce dernier étant équipé d'un circuit homologue de démultiplexage/multiplexage.

Le boîtier d'interfaçage 22 comprend une interface proprement dite 24 permettant de recevoir les signaux en provenance des électrodes RA, RV, LV et V+, ou d'émettre des impulsions de stimulation sur ces mêmes conducteurs. Un étage 26 permet de filtrer et d'amplifier les divers signaux de dépolarisation détectés ainsi que le signal EA. Un multiplexeur 28 assure par ailleurs la commutation des divers conducteurs sélectivement avec un système 30 de stimulation externe PSA (*Pacemaker System Analyser*, analyseur de système stimulateur), éventuellement inclus dans le boîtier d'interface 22, destiné à assurer de façon continue les fonctions de détection et de génération d'impulsions de stimulation pendant le test des différentes configurations, en relais du générateur implanté 10 ou avant le raccordement de celui-ci aux sondes 12, 16, 18 et 20.

L'ensemble comprend également un programmateur 40 ou autre système informatique, comprenant un module logiciel permettant l'acquisition et le traitement des signaux EA et de dépolarisation cardiaque, pour notamment le calcul des indices composites *Ind1* à *Ind4*, caractéristiques de l'invention. Ce programmateur comprend une unité de traitement 42, une unité de mesure 44 et une mémoire de stockage 46. Il peut être prévu éventuellement un clavier 48 ou autre moyen d'introduction de données. Enfin, un afficheur 50 permet de présenter au praticien les différentes possibilités qui lui sont offertes pour la définition des diverses configurations de stimulation. L'affichage peut notamment prendre la forme illustrée Figure 4, avec une représentation schématique des différentes régions du coeur identifiant les cibles principales :
- pour l'oreillette droite RA : appendice atrial AA, paroi latérale LW et septum atrial AS ;
- pour le ventricule droit RV : région de sortie OFD, septum bas, moyen et haut LS, MS et HS, et sommet APEX ;
- pour le réseau coronaire CS (par lequel sera stimulé le ventricule gauche) : grande veine cardiaque GCV, veine latérale LV, veine postérolatérale PLV, veine postérieure PV et veine cardiaque médiane MCV.

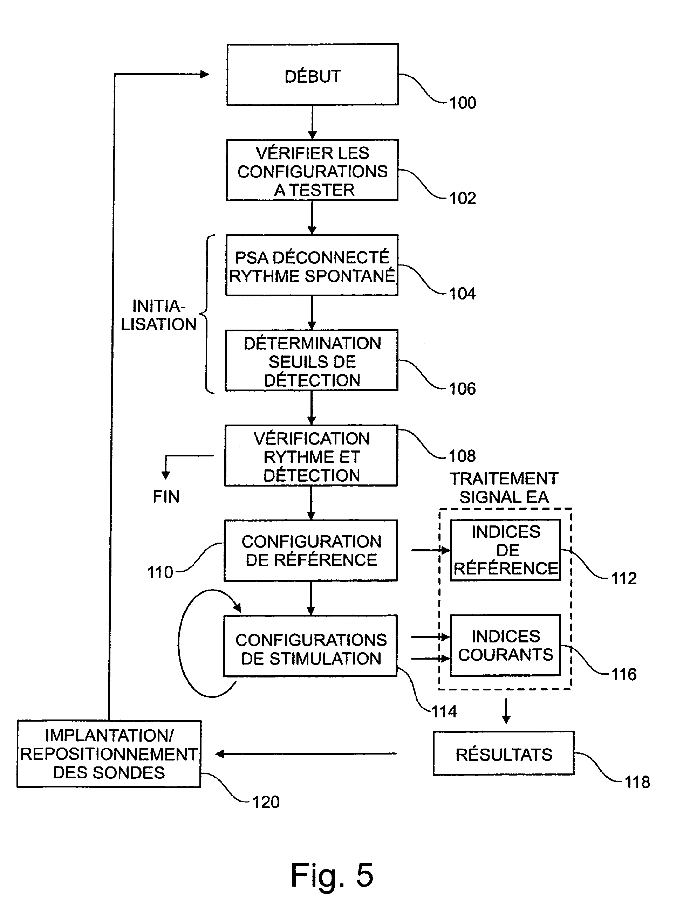

La Figure 5 illustre un exemple de mode opératoire, pour la sélection de la meilleure configuration de stimulation au moment de l'implantation. Après les différentes initialisations (étape 100), le praticien vérifie les configurations à tester (étape 102).

La condition de référence qui sera ensuite utilisée pour les diverses comparaisons peut être notamment celle correspondant au rythme spontané du patient de manière à laisser s'exprimer ce rythme spontané (étape 104). Par sécurité, le stimulateur externe PSA est normalement déconnecté des sondes, il sera connecté seulement pour les étapes ultérieures de test des diverses configurations de stimulation.

Les seuils de détection pour la synchronisation des signaux du capteur sont également déterminés lors de cette phase initiale (étape 106).

Le générateur externe PSA est ensuite connecté, une stimulation est appliquée puis diverses vérifications sont effectuées, notamment la stabilité du rythme (étape 108).

Une première configuration est ensuite testée (étape 110), le traitement du signal EA produisant (bloc 112) les indices composites *Ind1, Ind2, Ind3* et/ou *Ind4* recherchés (l'obtention de deux de ces indices étant généralement suffisant).

D'autres configurations de stimulation sont ensuite testées, avec des commutations d'électrodes différentes (étape 114). On notera que le paramétrage des DAV et DVV n'est généralement pas modifié à ce stade (seulement dans une phase ultérieure d'optimisation éventuelle après sélection des sites, et de suivi du patient). On peut notamment prévoir un DAV court de 80 à 100 ms environ, et un DVV nul.

Les indices correspondants sont évalués (bloc 116) et comparés aux indices de référence, pour donner un résultat global (étape 118).

Le praticien peut alors modifier l'implantation des sondes ou repositionner celles-ci (bloc 120) et réitérer le processus décrit plus haut (retour à l'étape 100), pour donner de nouveaux résultats à l'étape 118.

Les résultats successifs ainsi obtenus sont ensuite comparés, de manière à déterminer quelle est la configuration de stimulation procurant l'efficacité optimale.

Le praticien peut par exemple tester les diverses configurations suivantes. La première série de configurations à tester sera un ensemble "standard" comprenant :
- rythme spontané (servant de référence),
- stimulation du seul ventricule droit RV (à des fins de diagnostic, ou bien comme configuration de référence),
- stimulation du seul ventricule gauche,
- stimulation biventriculaire conventionnelle (qui peut également être choisie comme configuration de référence), avec stimulation concomitante des deux ventricules droit et gauche RV-LV.

Le diagnostic permet d'évaluer la sensibilité des indices dans les plus mauvaises conditions de stimulation, pour un patient en insuffisance cardiaque ; il ne s'agit toutefois jamais de considérer une telle stimulation comme une configuration de stimulation programmable possible.

Ensuite seront testées toutes les configurations possibles impliquant un repositionnement de sonde ou l'addition d'une sonde supplémentaire V+. Le repositionnement des sondes RV et LV implique de répéter le test pour les configurations de base RV seul, LV seul, et RV-LV.

L'ajout d'une sonde V+ additionnelle pour stimuler le ventricule gauche ou droit impliquera de tester toutes les combinaisons de stimulation possibles :
- biventriculaire (stimulation concomitante LV-V+ ou RV-V+),
- stimulation triventriculaire (stimulation concomitante V+, RV et LV),
- stimulation multiventriculaire (stimulation concomitante sur plusieurs électrodes d'une sonde multi-électrodes, et sur RV et LV).

La configuration en rythme spontané sera toujours réévaluée au début de chaque série de tests, et les indices seront comparés à ceux obtenus la première fois (configuration de référence). Si une variation substantielle de l'indice dans la condition spontanée est avérée, un message d'alerte sera affiché, et le praticien devra vérifier l'état du patient : en effet, une variation de l'indice ne devrait normalement pas apparaître, sinon il s'agit d'un symptôme de détérioration de l'état du patient, qu'il convient de prendre immédiatement en compte.

Le praticien peut par exemple tester les trois ensembles de configurations suivants :

| ensemble de configurations | Sondes implantées | Modifications apportées | configurations à tester (nouvelles ou modifiées) |
|---|---|---|---|
| n° 1 | RA, RV, LV | - | spontané, LV seul, BiV |
| n° 2 | RA, RV, LV | repositionnement RV | spontané, BiV |
| n° 3 | RA, RV, V+, LV | ajout V+ | spontané, BiV+, TriV |

Parmi toutes les configurations testées, celle qui sera sélectionnée sera celle produisant la valeur optimale du ou des indices composites (la valeur minimale avec les indices *Ind1* à *Ind4* exemplifiés plus haut).
Ultérieurement, une fois que les sites de stimulation auront été choisis, un algorithme d'analyse comparable pourra être mis en oeuvre pour tester des configurations différant par les paramétrages du DAV et/ou du DVV de manière à optimiser les valeurs de ces paramètres. Il pourra en être de même après implantation, dans le cadre du suivi du patient, de manière à évaluer l'incidence d'une modification de la configuration de stimulation et/ou du paramétrage du DAV et du DVV.

Il est ainsi possible de calculer les indices pour chaque couple de valeurs {DAV, DVV}, puis de choisir le couple qui est associé à la meilleure valeur d'indice. L'analyse pourra se faire en tentant d'optimiser les indices composites *Ind1, Ind2, Ind3* et/ou *Ind4* décrits plus haut (l'optimisation portant typiquement sur deux de ces indices).

Il est également possible d'utiliser les indices pour suivre l'évolution de la pathologie du patient sur le long terme, de manière à évaluer les effets délétères d'un remodelage et empêcher la survenue d'un accident consécutif à l'aggravation de l'état d'insuffisance cardiaque. Il s'agit alors d'évaluer l'évolution dans le temps d'un indice donné pour détecter une détérioration de l'état du patient et adresser un message d'alerte au praticien afin par exemple de modifier la thérapie (optimisation de la configuration de stimulation, reparamétrage des DAV et DVV, traitement médicamenteux, etc.).

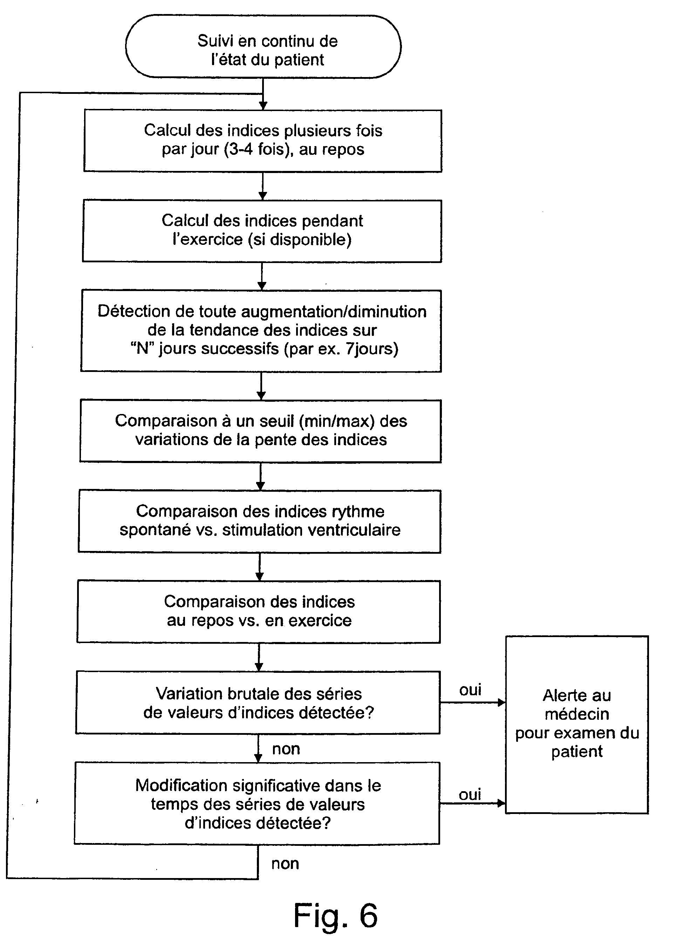

La figure 6 expose les diverses étapes d'un tel suivi du patient sur le long terme par analyse de l'évolution d'un ou plusieurs des indices *Ind1, Ind2, Ind3* et/ou *Ind4* décrits plus haut.

## Revendications

1. Un ensemble pour la recherche d'une configuration optimale de stimulation au moyen d'un dispositif médical actif (10) de resynchronisation par stimulation bi-, tri- ou multiventriculaire, cet ensemble comprenant :
- des moyens de stimulation ventriculaire (10 ; 30), aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante prédéterminée et modifiable ;
- un capteur d'accélération (14) apte à délivrer un signal d'accélération endocardiaque (EA) représentatif des contractions et relaxations cycliques du coeur ; et
- des moyens aptes à isoler et prétraiter, dans le signal d'accélération endocardiaque (EA) considéré sur un cycle cardiaque compris entre deux événements ventriculaires successifs : (i) une composante (EA1) correspondant au premier pic d'accélération endocardiaque, associé à la contraction ventriculaire isovolumique, et (ii) une composante (EA2) correspondant au second pic d'accélération endocardiaque, associé à la relaxation ventriculaire isovolumique,
cet ensemble comprenant en outre des moyens (40) évaluateurs de l'efficacité de ladite configuration de stimulation courante, comprenant :
- des moyens pour extraire au moins deux paramètres caractéristiques des composantes correspondant au premier pic d'accélaration endocardiaque (EA1) et correspondant du deuxième pic d'accélération endocardiaque (EA2) isolées et prétraitées :
- des moyens pour combiner lesdits au moins deux paramètres en un indice composite représentatif de l'efficacité de ladite configuration de stimulation courante ;
- des moyens pour déterminer une pluralité de valeurs dudit indice composite pour une pluralité correspondante de configurations de stimulation différentes ; et
- des moyens pour déterminer une configuration de stimulation préféren, tielle à partir de ladite pluralité de valeurs dudit indice composite, par recherche d'un optimum dudit indice composite,
**caractérisé en ce que** :
- lesdits au moins deux paramètres caractéristiques des composantes correspondant au premier pic d'accélaration endocardiaque (EA1) et correspondant du deuxième pic d'accélération endocardiaque (EA2) sont des paramètres du groupe formé par :
• *PEA1* = valeur de l'amplitude crête-à-crête de la composante correspondant au premier pic d'accélaration endocardiaque (EA1) ;
• *TstEA1* = durée d'apparition du début de la composante correspondant au premier pic d'accélaration endocardiaque (EA1), représentée par l'intervalle de temps séparant i) un marqueur temporel de début de cycle cardiaque et ii) le franchissement d'un seuil d'enveloppe d'énergie de la composante correspondant au premier pic d'accélaration endocardiaque (EA1) ;
• *LargEA1* = intervalle de temps séparant i) ledit franchissement du seuil d'enveloppe d'énergie de la composante correspondant au premier pic d'accélaration endocardiaque (EA1) de ii) l'instant du pic de ladite enveloppe d'énergie de la composante correspondant au premier pic d'accélaration endocardiaque (EA1); et
• *Syst* = durée de la systole, représentée par l'intervalle de temps séparant le *début* (*TstEA1)* de la composante correspondant au premier pic d'accélaration endocardiaque (EA1) du début (*TstEA2)* de la composante correspondant du deuxième pic d'accélération endocardiaque (EA2),
- **en ce que** ledit indice composite est un indice du groupe formé par :
- *Ind*1 = *(TstEA1 x LargEA1)* / *(Syst x PEA 1) ;*
• *Ind2* = *(TstEA1 x LargEA1)* / *[(Syst - LargEA1) x PEA1] ;*
- *Ind3* = *(TstEA1 x LargEA1)* / *(Syst) ;* et
- *Ind4 = (TstEA1)* / *(PEA*1),
- et **en ce que** ledit optimum est un minimum de cet indice.

2. L'ensemble de la revendication 1, comprenant :
- une sonde auriculaire (18) portant ledit capteur d'accélération (14) ; et
- des sondes ventriculaires droite (16) et gauche (18) portant à leur extrémité distale des électrodes aptes à définir respectivement lesdits sites de stimulation ventriculaire droit(s) et gauche(s) selon ladite configuration de stimulation courante.

3. L'ensemble de la revendication 2, comprenant en outre :
- une sonde ventriculaire additionnelle (20 ; 20'), droite ou gauche, portant à son extrémité distale des électrodes aptes à définir au moins un site de stimulation ventriculaire additionnel, définissant ladite configuration de stimulation courante en combinaison avec lesdits sites de stimulation ventriculaire droit(s) et gauche(s).

4. L'ensemble de la revendication 1, comprenant :
- ladite prothèse cardiaque implantable (10) de resynchronisation par stimulation bi-, tri- ou multiventriculaire ;
- une sonde auriculaire (18) portant ledit capteur d'accélération (14) ;
- des sondes ventriculaires droite (16) et gauche (18) portant à leur extrémité distale des électrodes aptes à définir respectivement lesdits sites de stimulation ventriculaire droit(s) et gauche(s) selon ladite configuration de stimulation courante ;
- éventuellement une sonde ventriculaire additionnelle (20 ; 20'), droite ou gauche, portant à son extrémité distale des électrodes aptes à définir au moins un site de stimulation ventriculaire additionnel, définissant ladite configuration de stimulation courante en combinaison avec lesdits sites de stimulation ventriculaire droit(s) et gauche(s) ;
- un équipement externe (30) de détection/stimulation ;
- un boîtier d'interfaçage (22) comprenant des moyens (26) de traitement dudit signal d'accélération endocardiaque (EA), ainsi que des moyens (28) pour coupler l'équipement externe auxdites sondes auriculaire et ventriculaires et
- des moyens programmateurs externes (40), couplés au boîtier d'interfaçage pour recevoir le signal d'accélération endocardiaque (EA) traité, et comprenant lesdits moyens évaluateurs de l'efficacité de la configuration de stimulation.

## Patentansprüche

1. Eine Anordnung für die Suche nach einer optimalen Stimulationskonfiguration mittels einer aktiven medizinischen Resynchronisationsvorrichtung (10) durch bi-, tri- oder multiventrikuläre Stimulation, wobei die Anordnung aufweist:
- Mittel zur ventrikulären Stimulation (10; 30), die geeignet sind, um Stimulationsimpulse auszugeben, die bestimmt sind, um auf implantierte Elektroden angewendet zu werden, jeweils in zumindest einem rechten ventrikulären Stimulationsgebiet und zumindest einem linken ventrikulären Stimulationsgebiet gemäß einer aktuellen vorbestimmten und modifizierbaren Stimulationskonfiguration;
- einen Beschleunigungssensor (14), der geeignet ist, um ein endokardiales Beschleunigungssignal (EA) auszugeben, welches für zyklische Anspannungen und Entspannungen des Herzens repräsentativ ist; und
- Mittel, die geeignet sind zum Isolieren und Vorverarbeiten in dem endokardialen Beschleunigungssignal EA bei Betrachtung während eines kardialen Zyklus zwischen zwei ventrikulären aufeinanderfolgenden Ereignissen: i) eine Komponente EA1, die einem ersten Höhepunkt endokardialer Beschleunigung entspricht, welcher dem ventrikulären isovolumetrischen Anspannen zugeordnet ist, und ii) eine Komponente EA2, die einem zweiten endokardialen Beschleunigungshöhepunkt entspricht, der einer ventrikulären isovolumetrischen Entspannung zugeordnet ist,
diese Anordnung, die weiterhin Evaluierungsmittel (40) der Effektivität der aktuellen Stimulationskonfiguration aufweist, weist auf:
- Mittel zum Extrahieren zumindest zweier charakteristischer Parameter der isolierten und vorverarbeiteten Komponenten, die dem ersten endokardialen Beschleunigungshöhepunkt EA1 entsprechen und die dem zweiten endokardialen Beschleunigungshöhepunkt EA2 entsprechen:
- Mittel zum Kombinieren der zumindest zwei Parameter in einem Verbundindex, der für die Effektivität der aktuellen Stimulationskonfiguration repräsentativ ist;
- Mittel zum Bestimmen einer Vielzahl von Werten des Verbundindex für eine entsprechende Vielzahl von unterschiedlichen Stimulationskonfigurationen; und
- Mittel zum Bestimmen einer bevorzugten Stimulationskonfiguration aus der Vielzahl von Werten des Verbundindex durch eine Suche nach einem Optimum des Verbundindex,
**dadurch gekennzeichnet, dass**:
- die zumindest zwei charakteristischen Parameter der Komponenten EA1 und EA2, die einem ersten endokardialen Beschleunigungshöhepunkt entsprechen, und die einem zweiten endokardialen Beschleunigungshöhepunkt entsprechen, Parameter aus einer Gruppe sind, die gebildet wird aus:
• *PEDA1* = Wert der Spitze-bis-Spitze-Amplitude der Komponente EA1, die einem ersten endokardialen Beschleunigungshöhepunkt entspricht;
• *TstEA1* = Dauer eines Auftretens eines Beginns der Komponente EA1, die einem ersten endokardialen Beschleunigungshöhepunkt entspricht, welche durch das Zeitintervall dargestellt wird, welches zwischen i) einem zeitlichen Marker des Beginns des kardialen Zyklus und ii) dem Überschreiten eines Schwellwerts einer Energieeinhüllenden der Komponente liegt, die einem ersten endokardialen Beschleunigungshöhepunkt entspricht;
• *LargEA1* = ein Zeitintervall, welches zwischen i) dem Überschreiten eines Schwellwerts einer Energieeinhüllenden der Komponente, die einem ersten endokardialen Beschleunigungshöhepunkt entspricht, und ii) dem Moment eines Höhepunkts der Energieeinhüllenden der Komponente EA1 liegt, die einem ersten endokardialen Beschleunigungshöhepunkt entspricht; und
• *Syst* = Dauer der Systole, die durch das Zeitintervall dargestellt ist, welches zwischen dem Anfang (*TstEA1*) der Komponente EA1, die dem ersten endokardialen Beschleunigungshöhepunkt entspricht und einem Beginn (*TstEA2*) der Komponente EA2, die einem zweiten endokardialen Beschleunigungshöhepunkt entspricht liegt,
- dadurch, dass der Verbundindex ein Index aus einer Gruppe ist, die gebildet wird aus:
• *Ind1* = *(TastEA1 x LargEA1)*/*(Syst x PEA1);*
• *Ind2* = *(TstEA1 x LargEA1)*/*[(Syst - LargEA1) x PEA1];*
• *Ind3* = *(TstEA1 x LargEA1)*/*(Syst); und*
• *Ind4* = *(TstEA1)*/*(PEA1),*
- **und dadurch,** dass das Optimum ein Minimum dieses Index ist.

2. Anordnung nach Anspruch 1, aufweisend:
- eine aurikulare Sonde (18), die den Beschleunigungssensor (14) trägt; und
- rechte (16) und linke (18) ventrikulare Sonden, die an ihrem distalen Ende Elektroden tragen, die geeignet sind, um jeweils die ventrikulären rechten und linken Stimulationsgebiete gemäß der aktuellen Stimulationskonfiguration zu definieren.

3. Anordnung nach Anspruch 2, weiterhin aufweisend:
- eine zusätzliche ventrikuläre Sonde (20; 20'), rechts oder links, die an ihrem distalen Ende Elektroden trägt, die geeignet sind, um zumindest ein zusätzliches ventrikuläres Stimulationsgebiet zu definieren, und die aktuelle Stimulationskonfiguration in Kombination mit den rechten und linken ventrikulären Stimulationsgebieten definieren.

4. Anordnung nach Anspruch 1, aufweisend:
- die implantierbare kardiale Resynchronisationsprothese (10) durch bi-, tri- oder multiventrikuläre Stimulation;
- eine aurikuläre Sonde (18), die den Beschleunigungssensor (14) trägt;
- ventrikuläre rechte (16) und linke (18) Sonden, die an ihrem distalen Ende Elektroden tragen, die geeignet sind, um jeweils die ventrikulären rechten und linken Stimulationsgebiete gemäß der aktuellen Stimulationskonfiguration zu definieren;
- eventuell eine zusätzliche ventrikuläre Sonde (20; 20'), rechts oder links, die an ihrem distalen Ende Elektroden trägt, die geeignet sind, um zumindest ein zusätzliches ventrikuläres Stimulationsgebiet zu definieren, um die aktuelle Stimulationskonfiguration in Kombination mit den ventrikulären rechten und linken Stimulationsgebieten zu definieren;
- eine externe Ausstattung (30) zum Erfassen/Stimulieren;
- eine Schnittstelleneinheit (22), die Mittel (26) zum Verarbeiten des endokardialen Beschleunigungssignals EA aufweist, ebenso wie Mittel (28) zum Koppeln der externen Ausstattung an die aurikularen und ventrikulären Sonden; und
- externe Programmiermittel (40), die mit einer Schnittstelleneinheit gekoppelt sind, um das verarbeitete endokardiale Beschleunigungssignal EA zu empfangen, und die Mittel zum Auswerten der Effektivität der Stimulationskonfiguration aufweist.

## Claims

1. A unit for finding an optimal configuration of stimulation by means of an active medical device (10) for resynchronization by bi-, tri- or multi-ventricular stimulation, the unit comprising:
- ventricular stimulation means (10; 30) adapted to deliver stimulation pulses intended to be applied to electrodes implanted in at least one right ventricular stimulation site and at least one left ventricular stimulation site, respectively, according to a predetermined and modifiable current configuration of stimulation;
- an acceleration sensor (14) adapted to deliver an endocardial acceleration signal (EA) representative of the cyclic contractions and relaxations of the heart; and
- means adapted to isolate and preprocess, in the endocardial acceleration signal (EA) considered over a cardiac cycle comprised between two successive ventricular events: (i) a component corresponding to the first peak of endocardial acceleration (EA1), associated with the isovolumic ventricular contraction, and (ii) a component corresponding to the second peak of endocardial acceleration (EA2), associated with the isovolumic ventricular relaxation,
the unit further comprising means (40) for evaluating the efficiency of said current configuration of stimulation, comprising:
- means for extracting at least two characteristic parameters of the isolated and preprocessed components corresponding to the first peak of endocardial acceleration (EA1) and corresponding to the second peak of endocardial acceleration (EA2);
- means for combining said at least two parameters into a composite index representative of the efficiency of said current configuration of stimulation;
- means for determining a plurality of values of said composite index for a corresponding plurality of different configurations of stimulation; and
- means for determining a preferential configuration of stimulation based on said plurality of values of said composite index, by searching for an optimum of said composite index,
**characterized in that**:
- said at least two characteristic parameters of the components corresponding to the first peak of endocardial acceleration (EA1) and corresponding to the second peak of endocardial acceleration (EA2) are parameters of the group formed by:
. *PEA1* = value of the peak-to-peak amplitude of the component corresponding to the first peak of endocardial acceleration (EA1);
. *TstEA1* = duration of occurrence of the start of the component corresponding to the first peak of endocardial acceleration (EA1), represented by the time interval separating i) a cardiac cycle start timestamp and ii) the crossing of a threshold of energy envelope of the component corresponding to the first peak of endocardial acceleration (EA1); and
. *LargEA1* = time interval separating i) said crossing of the threshold of energy envelop of the component corresponding to the first peak of endocardial acceleration (EA1) from ii) the instant of the peak of said energy envelop of the component corresponding to the first peak of endocardial acceleration (EA1); and
. *Syst* = duration of the systole, represented by the time interval separating the start (TstEA1) of the component corresponding to the first peak of endocardial acceleration (EA1) from the start (TstEA2) of the component corresponding to the second peak of endocardial acceleration (EA2),
- **in that** said composite index is an index of the group formed by:
. *Ind1* = *(TstEA1 x LargEA1)* / *(Syst* x *PEA1);*
. *Ind2* = *(TstEA1 x LargEA1)* / *[(Syst - LargEA1) x PEA1)];*
. *Ind3* = *(TstEA1 x LargEA1)* / *(Syst); and*
. *Ind4* = *(TstEA1)* / *(PEA1),*
- and **in that** said optimum is a minimum of this index.

2. The unit of claim 1, comprising:
- an atrial lead (18) carrying said acceleration sensor (14); and
- right (16) and left (18) ventricular leads carrying at their distal ends electrodes adapted to define said at least one right and left ventricular stimulation sites, respectively, according to said current configuration of stimulation.

3. The unit of claim 2, further comprising:
- a right or left additional ventricular lead (20; 20') carrying at its distal end electrodes adapted to define at least one additional ventricular stimulation site, defining said current configuration of stimulation in combination with said at least one right and left ventricular stimulation sites.

4. The unit of claim 1, comprising:
- said implantable cardiac prosthesis (10) for resynchronization by bi-, tri- or multi-ventricular stimulation;
- an atrial lead (18) carrying said acceleration sensor (14);
- right (16) and left (18) ventricular leads carrying at their distal ends electrodes adapted to define said at least one right and left ventricular stimulation sites, respectively, according to said current configuration of stimulation;
- possibly a right or left additional ventricular lead (20; 20') carrying at its distal end electrodes adapted to define at least one additional ventricular stimulation site, defining said current configuration of stimulation in combination with said at least one right and left ventricular stimulation sites;
- an external detection/stimulation equipment (30);
- an interface box (22) comprising means (26) for processing said endocardial acceleration signal (EA), as well as means (28) for coupling the external equipment to said atrial lead and ventricular leads; and
- external programing means (40), coupled to the interface box to receive the processed endocardial acceleration signal (EA) and comprising said means for evaluating the efficiency of the configuration of stimulation.
